# EUROPEAN PATENT SPECIFICATION

(11) **EP 1 390 091 B1**
(45) Date of publication and mention of the grant of the patent: **14.01.2009**
(21) Application number: 02730420.3
(22) Date of filing: 27.05.2002
(51) Int. Cl.: A61M 15/00

(54) **INHALER**
INHALATOR
DISPOSITIF

(30) Priority: 26.05.2001 GB 0112888
(43) Date of publication of application: 25.02.2004
(73) Proprietor: Innovata Biomed Limited, St. Albans AL1 3HW (GB)
(72) Inventor: BRAITHWAITE, Philip, Tewkesbury GL20 8NB (GB)
(74) Representative: Jones, Stephen Anthony
(86) International application number: PCT/GB2002/002251
(87) International publication number: WO 2002/096489

(56) References cited:
- WO-A-90/07351
- WO-A-99/13930
- US-A- 5 458 135
- US-A- 5 653 227
- US-A- 5 785 049

## Description

This invention relates to a novel air amplifying system and a novel powder delivery device comprising such a system, for example, a medicament delivery device, such as an inhaler.

In particular the invention provides a novel form of dry powder inhaler and a method of delivering a powder using such an inhaler.

Conventional powder delivery devices, such as dry powder inhalers (DPIs), deliver a powder dosage by the aerosolisation of the powder, the aerosolisation being largely driven by the inhalation of the patient. One disadvantage with these conventional DPIs is that the extent of aerosolisation, and therefore the consistency of the dosage delivered, is dependent upon, *inter alia,* the inspiratory flow of the patient, the nature of the air passage and the nature of the formulation.

Attempts have been made to improve on conventional DPIs by using, for example, an air jet directed at or across a powder. However, such systems suffer from a number of disadvantages in that, *inter alia,*
(i) A powder container may be difficult to completely empty, giving rise to problems with dosage consistency and with efficiency of delivery. There may also be a lack of any real element of control of the air stream.
(ii) There is no amplification, i.e., the volume of air entering the device is the same as the volume of air leaving the device, which may limit the efficiency of powder aerosolisation.
(iii) Air flowing across the powder due to the inhalation of a patient can only lift the powder into the airstream and therefore does not efficiently aerosolise the powder.

Conventional metered dose inhalers (MDIs) attempt to address this problem by the use of a volatile propellant to create a pressure sufficient to aerosolise the medicament. However, one disadvantage of MDIs is that the combination of a volatile propellant to create a pressure sufficient to aerosolise the medicament, and a solubilised medicament can give rise to blocking or clogging of the valve through which the aerosolised medicament is emitted. In addition MDIs are disadvantageous in that, *inter alia,* they lack the ability to co-ordinate actuation with inhalation, and suffer from high drug impaction in the oropharynx, although breath actuation systems may overcome these issues to a certain extent.

US Patent No 6,158,675 to Nathaniel Hughes, describes a microatomising device which uses a vortex accumulation resonant chamber the use of which creates a vacuum to enable outside entrainment air to be drawn into the device, lowering the speed of delivery of the medicament particles to the lung.

US Patent No. 4,114,615 to Draco AB, describes an aerosol inhalation device which, *inter alia,* activates a liquid propellant. In use, the propellant flows past a capillary arranged in a medicament container. The specification describes, at column 3, lines 59 to 63, that when the propellant passes across the top of the capillary, the medicament is drawn from the chamber.

US Patent No. 5,657,794 to Inhale Therapeutics Inc. describes a dry powder inhaler which is provided with a curved section of a passage which creates a Venturi effect to empty a powder containing receptacle. A feed tube is positioned so that an inlet end of the tube enters the receptacle and a high velocity gas stream is released which creates a 'low pressure region' at the outlet end of the feed tube. This low pressure region that is created acts to draw fluidisation air into the receptacle, to fluidise and/or aerosolise the powder and extract the powder through the feed tube and into the high velocity gas stream. Although the device addresses the problem of more complete emptying of the powder receptacle by the utilisation of a walled passage which communicates and co-operates with a depression in the powder receptacle to create a Venturi effect, such a device may, *inter alia,* have limitations in efficiency of powder aerosolisation and may not address all previously mentioned problems of prior art devices.

In addition, one particular disadvantage of the Inhale device is its large size. The Inhale system generates a substantially laminar flow of, e.g. powder. Thus, in order to achieve the necessary deagglomeration, a large dose of high impact air must be delivered in order to achieve the magnitude of impact required for deagglomeration of the powder. Thus, the Inhale system suffers from the disadvantage that, *inter alia,* is cumbersome and does not readily lend itself to a portable delivery system.

International Patent application No. WO 01/87378 to Dura, describes a dry powders inhaler wherein a powder port extends into a dispersion tube. A small burst of compressed gas is released into the dispersion tube and expands; the rapidly moving and expanding gas disperses the powder and entrains the powder in the gas flow. However, the device suffers from the disadvantage that, *inter alia,* deagglomeration of the powder remains unsatisfactory.

US 5,740,794 - Inhale describes an inhalation apparatus which comprises, *inter alia,* a powder containing receptacle. A feed tube is positioned so that an inlet end of the tube enters the receptacle and a high pressure gas stream is released which creates a 'low pressure region' at the outlet end of the feed tube. This low pressure region acts to draw fluidisation air into the receptacle, to fluidise and extract the powder through the feed tube and into the high velocity gas stream.

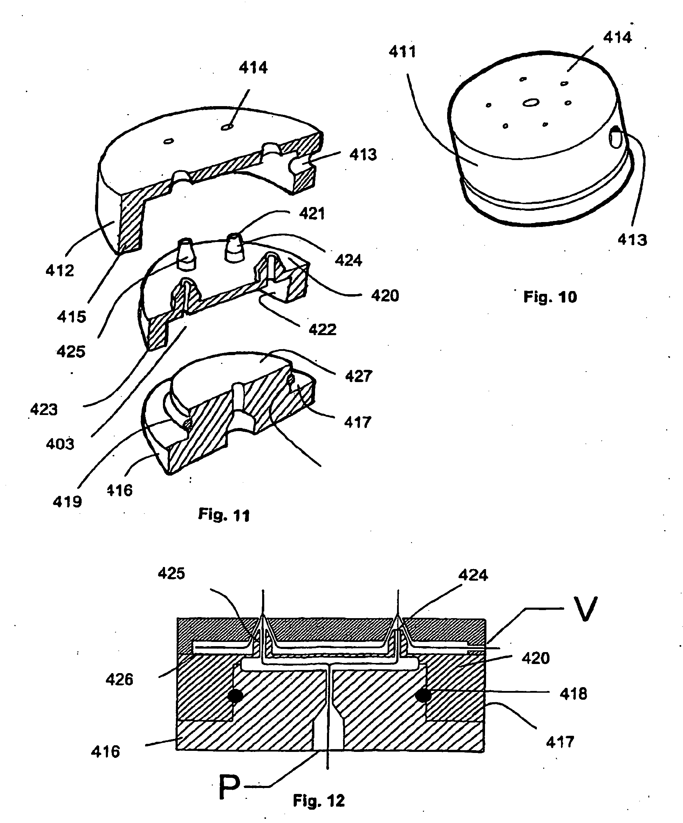

However, there is no disclosure that the powder will undergo a circulatory trajectory on its way to the mouthpiece. Indeed, the disclosure, for example in Fig. 12 of Inhale describes a system wherein the powder is evacuated from the receptacle through a 'central' feed tube, no substantial circulatory motion being introduced.

Furthermore, the description refers to an "undisrupted" flow path for the powder, which would lead one to conclude that a "feed tube" which is central rather, than one which is peripheral, is desirable.

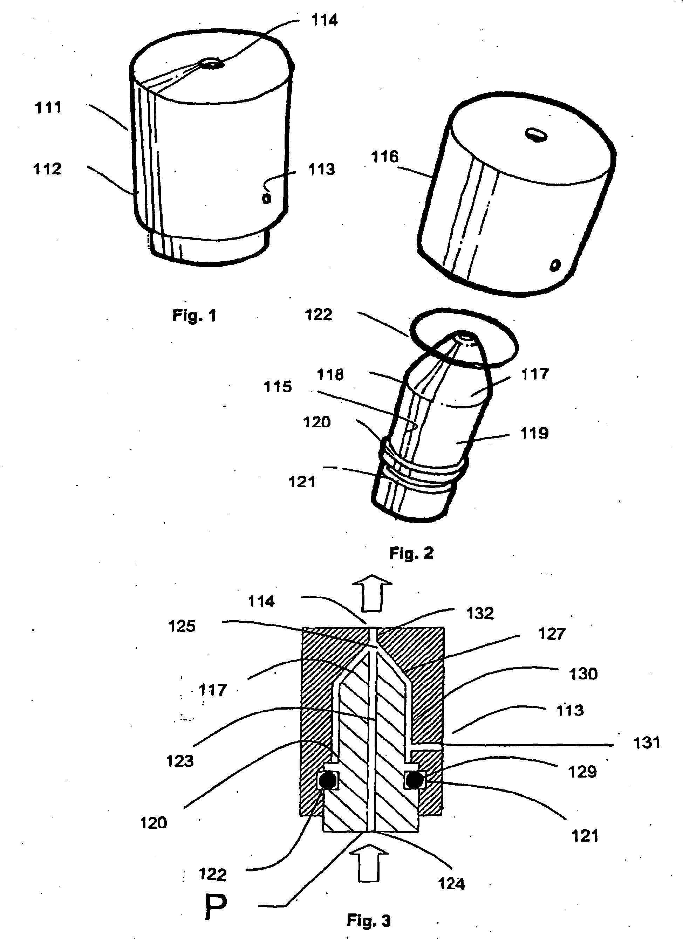

International Patent Application No. WO 00/45878 - Fraunhofer describes a device which utilises a vacuum aerosolisation of a liquid/powder. However, it is notable, particularly from Figure 2, that the powder/liposome travels through the central conduit with compressed air circulating around the outside of the conduit.

Thus there has long been a need for a powder delivery system which is capable of overcoming the aforementioned disadvantages. Attempts have been made to improve the respirable fraction of a powder (FPF) but these generally comprise the use of very low density particles. For example US Patent No. 6,254,854 describes the use of particles with a density of less than 0.4g cm⁻³, whereas conventional particles in powders administered, e.g. by inhalation, may have a density of about 0.8 to 1gcm⁻³.

Thus, there is clearly a need for the development of a device suitable for the delivery of particles of any density, which provides low powder retention and provides a high respirable dose. For example, particles of conventional density, or low density particles as hereinbefore described, or even higher density particles.

WO 90/07351 discloses an oral inhaler including a pressure gas source or pumping device for briefly providing a vigorous gas flow directed towards a mixing chamber. The two part form of claim 1 is based on the disclosure of WO 90/07951. US 5,785,049 discloses a method of aerosolizing a powdered medicament that involves drawing the powder through a tube and dispersing it in a high pressure gas stream. US 5,458,135 discloses a device for delivering aerosolized medicaments which involves dispersing a measured amount of medicament in a measured volume of a carrier gas. WO 99/13930 discloses a device for the intranasal administration of medicament, wherein the medicament is placed in a cavity into which air is drawn through air inlet slots. US 5,653,227 discloses a method of dispensing a flowable material in which a particulate or liquid material is entrained in a flow of gas and atomised.

We have now developed a powder delivery system which may comprise a number of high efficiency, controllable, elements and therefore overcomes or mitigates the disadvantages of the prior art. In particular the powder delivery system of the present invention overcomes the problem of MDIs by separation of the propellant volatile fluid and the powder. Furthermore, the powder delivery system of the invention overcomes the problems associated with prior art DPI devices and, *inter alia,* provides a greater efficiency of aerosolisation. It is therefore especially suited for use as a portable or hand held delivery device.

The invention is defined in claim 1. Preferred embodiments are defined in dependent claims 2- 56.

Thus according to a first feature of the invention we provide an air amplifying system comprising an amplifying fluid jet provided with a fluid inlet and a fluid outlet, the fluid outlet being linked to an outlet nozzle via an amplifying passage, the amplifying passage also being linked to a powder chamber, said chamber being adapted for non-laminar powder flow, such that fluid travelling from the fluid outlet of the jet draws extraneous air and aerosolised powder through the powder chamber so that the extraneous air and aerosolised powder mix with the amplifying fluid in the amplifying passage and the amplified mixture exits through the outlet nozzle characterised in that the powder chamber is an annular chamber which is adapted to provide non-laminar flow of the powder.

In particular, the air amplifying system of the invention utilises an amplified fluid, e.g. gas, stream to disperse a powder. An unamplified gas stream can be created which is of sufficient velocity, for example by passing through an amplifying jet, so that, as it exits the jet and passes across an amplification passage, in the form of a first opening of a contiguous powder chamber or conduit, the gas stream creates a vacuum in the contiguous chamber or conduit.

The chamber or conduit can be provided with a powder reservoir or a powder metering member adjacent an inlet to the powder chamber, such that the vacuum created by the exit of the gas stream from the amplifying jet creates a vacuum in the powder chamber and an entrainment air flow through the powder.

This entrainment air flow is sufficient to cause deagglomeration and/or entrainment and then subsequent aerosolisation of the powder. One effect of the vacuum is to deagglomerate the powder without direct impingement of the gas stream on the powder. This may limit impaction of powder and hence retention of powder within the device which may be a problem with some prior art devices. Moreover, the gas stream can also be adapted to be deflected against a solid surface, the effect being the tendency of the flow to become attached to or flow around the solid surface. The exploitation of this effect, therefore enables a 'shape' to be given to the existing gas stream. One advantage of the system of the invention is that, *inter alia,* it provides a greater efficiency of deagglomeration and/or aerosolisation over prior art devices by the direction of the entrained air.

We have especially found that by influencing the shape of the gas stream to have a substantially non-laminar motion provides an improvement in the deagglomeration of the powder reflected in a significant improvement in respirable or fine particle fraction (FPF) of the delivered powder aerosol and a reduction in the powder retention within the device. Furthermore, Computational Fluid Dynamics (CFD) studies indicate significantly improved fluid dynamics.

In the invention the powder chamber is adapted such that the aerosolised powder is deliberately subjected to a non-laminar flow by the use of an annular powder chamber. Thus, the air amplification system of the invention is provided with an annular powder chamber and an axial fluid jet.

Thus, in one embodiment of the invention the powder chamber may substantially form the body of the amplification system or be circumferential to the body of the device and the amplifying fluid jet may be axial to the body. In this particular embodiment the powder chamber may be a thin annular chamber. Preferably, the thin annular chamber may be created by bringing together male and female portions. Therefore, the outlet end of the fluid jet may comprise, or alternatively, may be fitted to, a frusto conical male member which fits into an outer portion of the powder chamber, e.g. in the form of a female member.

In this embodiment of the invention the separation between the male and female members may vary. Preferably, the separation between the male and female members which may be identified as the clearance may be from 100 to 5000 µm, preferably from 500 to 2000 µm. Most preferably, the clearance may be about 1000 µm.

Thus, the diameter of the jet may be from 100 to 500 µm, preferably from 200 to 300 µm, most preferably 250 µm. The diameter of the nozzle may vary, but may be from 100 to 1500 µm, preferably from 400 µm to 1200 µm, especially from 400 µm to 600 µm, e.g. 500 µm.

In the air amplifying system of the invention the dimensions of the nozzle and jet may vary depending, *inter alia,* upon the nature of the powder to be delivered. However, importantly, the nozzle should possess a greater diameter than that of the diameter of the jet. This particular aspect of the invention is advantageous in that as the fluid, e.g. air, leaves the jet through the nozzle it expands creating a vacuum in the adjacent powder chamber. Thus, the ratio of the diameter of the jet to the diameter of the nozzle may vary, but may be in the range of from 1:8 to 1:2, preferably 1:4 to 1:2 and especially 1:2. Furthermore, in the air amplifying system of the invention the shape of the nozzle may be changed and/or multiple nozzles may be used to, *inter alia,* reduce oropharyngeal deposition. A particular advantage of the present invention is that, *inter alia,* the air amplifying system has the ability to "slow" the aerosol. Conventionally known inhalers require the use of, for example, a spacer tube to achieve this. Thus, the air amplifying system can `slow' the aerosol without the use of such a spacer tube.

The powder reservoir and/or metering member may be contiguous with the powder chamber. Alternatively, the powder chamber may be connected to the powder reservoir and/or metering member by one or more conduits.

The air amplifying system of the invention may be useful in a variety of situations. However, it is especially useful when incorporated in a powder delivery device.

Thus according to a second feature of the invention we provide a powder delivery device which comprises a delivery passage, a powder reservoir and/or a metering member adapted to present a measured dose of powder to the delivery passage characterised in that the powder delivery device is provided with an air amplification system as hereinbefore described.

In a particularly preferred embodiment of the invention, the air amplifying system creates an entrained air flow through the powder reservoir and/or metering member. Thus, the powder reservoir and/or metering member, may be positioned adjacent a powder inlet and the flow through the amplifying jet is sufficient to draw entrained air and powder through the inlet. The reservoir and the metering member may be separate, e.g. a bulk powder reservoir with a metering member. Alternatively the reservoir and metering member may comprise a single item, thus, for example, the device of the invention may be provided with one or a plurality of prefilled metering members.

It should be understood that the basis of this aspect of the present invention is the creation of a pressure differential across or through the powder reservoir and/or metering member which enables the deagglomeration of the powder to occur, Therefore, the creation of a pressure differential may generally comprise the creation of a vacuum. It is especially preferred that the entrained air will flow through the powder which is presented either direct from the reservoir or, preferentially from the metering member. Thus, preferably, the entrained air inlet will be positioned adjacent to a first side of the reservoir and/or metering member and the vacuum is created adjacent a second, opposite side of the reservoir and/or metering member. In a further embodiment, a further inlet tube may be provided which is adapted to introduce entrainment air, e.g. flushing air into the reservoir/metering member.

It is further preferred that the entrained air flow is sufficient to both deagglomerate and aerosolise the powder, although, as hereinbefore described, *inter alia,* improved deagglomeration can be achieved by the use of a non-laminar entrained air flow.

The air amplifying system of the invention may be used in conjunction with a variety of delivery devices. However, the powder delivery system is especially suited for use in the delivery of a powdered medicament. Such a system may be used for the delivery of any type of powdered medicament, but the system finds particular utility in the delivery of an inhaled medicament. Thus the system of the invention may be used as or in conjunction with an inhaler, e.g. a dry powder inhaler.

Thus according to a preferred aspect of the invention the powder delivery device of the invention may be an inhaler. We especially provide a dry powder inhaler characterised in that it incorporates a powder delivery device as hereinbefore described.

In a further embodiment of the invention the amplification system may be provided with a plurality of nozzles and/or a plurality fluid jets. Such a plurality of nozzles and/or jets may increase the volume of powder which may be drawn the powder chamber. At the same time the total velocity of the fluid flowing through the jets and/or exiting through the nozzle. This is especially advantageous in the case of delivery of a powdered medicament, e.g. in an inhaler, since it enables a low velocity aerosolised powder cloud to be generated. In a yet further embodiment the fluid jet may comprise a plurality of interlocking jets. In such a case each jet may, optionally, be provided with one or more powder inlets. Furthermore, the system may be arranged to provide the separate, sequential or simultaneous operation of the jets to enable the creation of an aerosolised powder which coincides with, for example, the inspiration of a patient.

When the vacuum means comprises a Venturi-type system as hereinbefore described the pressurised fluid may be any fluid moving system. The fluid may be a liquid, however, preferentially, the fluid is a gas, for example, compressed air or a gas/vapour generated from the volatilisation of a volatile propellant, such as that delivered from a pressurised canister. Alternatively, the fluid flow may be generated by an electric motor, e.g. a battery operated motor, or by a manually primed piston, e.g. a hand pump.

When the vacuum means comprises the use of a volatilised propellant, any conventionally known pharmaceutically and/or environmentally acceptable propellants may be utilised. Such propellants include, but are not limited to, non-CFC propellants, such as a hydrofluoroalkane (HFA). Any conventionally known HFA propellant may be used, including those disclosed in, for example, EP0372777, WO91/04011, WO91/1117 WO91/11495 and WO91/14422. However, the most preferred HFA is a fluoroalkane such as a fluoromethane or a fluoroethane or a mixture of fluoroalkanes. Such fluoroalkanes include, but are not limited to, trichlorofluoromethane, dichlorodifluoromethane, 1,2-dichlorotetrafluorethane, trichlorotrifluoroethane and chloropentafluoroethane. One HFA which may be mentioned is HFA 134 (1,1,1,2-tetrafluoroethane) or HFA 227.

When the delivery device of the invention is utilised as or in conjunction with an inhaler, it is especially advantageous utilisation of entrained air not only deagglomerates the powder but also helps to facilitate aerosolisation of the powder.

When the powder delivery device comprises an inhaler, it may comprise a conventionally known inhaler with a system of the invention attached thereto. An example of a conventional inhaler is a CLICKHALER (available from Innovata Biomed in the UK and described in European Patent application No. 0 539 469) which is provided with an inhalation passage. The delivery device of the invention may optionally be attached, for example, at the outlet end of such an inhaler, to a spacer device.

In one embodiment, the metering member is adapted to transfer measured doses of powder from the powder reservoir to the delivery passage.

However, in an alternative embodiment, the powder may be presented to the delivery passage in a closed form, wherein it is opened in the delivery passage. Thus, the metering member may be a capsule, in which case the device may optionally be provided with means for piercing or rupturing the capsule.

In a yet further and preferred embodiment the powder may be presented to the delivery passage in an open form. Thus, for example, the metering member may be a spool carrying a powder, in which case the device may be provided with means for presenting the spool, in an open form into the delivery member.

Thus, the metering member may comprise a spool housed in a spool carrier. Such spools are generally described in the prior art. An example of such an inhaler system is a TECHNOHALER (available from Innovata Biomed in the UK and described in European Patent Application No. 0 626 689). Each spool has a flange at each end which form a tight slidable fit within the body of the spool carrier. The space left between the body of the spool and the spool carrier is filled with an appropriate powder. In an alternative embodiment the delivery device may be provided with a spool chamber, for example, in the form tube adjacent the delivery passage. In a preferred embodiment the spool chamber may form a snug fit around the spool and may therefore replace the spool carrier. The spool chamber may therefore optionally be fitted with an actuator member which may comprise a push rod mechanism.

The delivery device of the invention is advantageous in that, *inter alia,* it may operate by the administration of a cloud of powder. The device provides a dry powder delivery system which is independent of the rate of inspiration of a patient, and without the need for a patient to inhale undesirable propellants.

Furthermore, the inhaler of the invention is especially advantageous in that, *inter alia,* it may provide a significant increase in the respirable fraction of a delivered powder. As hereinbefore described, it is a particular aspect of the inhaler of the present invention that the inhaler may not require the use of a spacer, but still be able to "slow" the aerosol.

A variety of powders may be administered by using the inhaler of the invention. Such powders are generally drugs for the treatment of asthma, chronic obstructive pulmomary disease and respiratory infections. Such powders include, but are not limited to β₂-agonists, e.g. fenoterol, formoterol, pirbuterol, reproterol, rimiterol, salbutamol, salmeterol and terbutaline; non-selective beta-stimulants such as isoprenaline; xanthine bronchodilators, e.g. theophylline, aminophylline and choline theophyllinate; anticholinergics, e.g. ipratropium bromide; mast cell stabilisers, e.g. sodium cromoglycate and ketotifen; bronchial anti-inflammatory agents, e.g. nedocromil sodium; and steroids, e.g. beclomethasone dipropionate, fluticasone, budesonide and flunisolide; and combinations thereof.

It is within the scope of this invention for two or more powders to be administered.

Specific combinations of powders which may be mentioned include combinations of steroids, such as, beclomethasone dipropionate, fluticasone, budesonide and flunisolide; and combinations of to β₂-agonists, such as, formoterol and salmeterol. It is also within the scope of this invention to include combinations of one or more of the aforementioned steroids with one or more of the aforementioned β₂-agonists.

Further powders which may be mentioned include systemically active materials, such as, proteinaceous compounds and/or macromolecules, for example, hormones and mediators, such as insulin, human growth hormone, leuprolide and alpha interferon; growth factors, anticoagulants, immunomodulators, cytokines and nucleic acids.

It is within the scope of this invention to include combinations of any of the aforementioned medicaments.

The particle size of the powder may be varied depending, *inter alia,* on the type of aerosol being formed. In the case of a dry powder medicament, the particle size of the powder, and the carrier, if one is present may be varied. The nature of the carrier may also be varied. Thus, the particle size of the powder may be substantially between 1 and 100 µm. That is, at least 90% w/w of the powder should have a particle size of between 1 and 100 µm. The preferred particle size may also depend upon the nature of the powder being delivered. Thus, for example, for the treatment of respiratory disorders a particle size of 4 to 8 µm may be preferred, e.g. 6 µm.

However, for the delivery of systematically active powders a smaller particle size may be desirable, for example from 1 to 5 µm, e.g. 2 µm.

In a dry powder formulation a variety of carriers may be used. Certain carriers may be mentioned, by way of example only, such as sugars, e.g. dextran, mannitol and lactose, for example α-lactose monohydrate. The particle size of the carrier may be across a wide range, between 0.1 and 500µm, preferably between 1 and 200 µm. Alternatively, the carrier may itself comprise a mixture of fine and coarse particles.

As previously mentioned the powder delivery device of the invention is especially suited for use as a medicament delivery device, e.g. an inhaler. Therefore, we further provide a method of treatment of a patient with a respiratory disorder which comprises the administration of a powdered medicament using a device as hereinbefore described. In an especially preferred embodiment the method comprises administration of medicament by inhalation.

The device of the invention is especially suited for the efficient delivery of macromolecules, such as insulin.

When the device of the invention is used for the delivery of macromolecules, such as insulin, it is important that they be provided in a moisture resistant system. Thus, according to the invention we provide a device as hereinbefore described provided with a moisture resistant coating e.g. a paraxylylene coating.

The device of the invention is advantageous in that, *inter alia,* a significantly increased respirable fraction is achieved. A conventional inhaler might be expected to deliver a respirable or fine particle fraction of, for example, 20 - 40%. However, the delivery device of the invention is able to provide an FPF of in excess of 70%.

The respirable fraction of a powder, known as FPF is generally a measurement of the percentage of a powder that reaches the lung of a patient as of function of the delivered dose. Respirable powder particles are considered to be about 6 µm (aerodynamic diameter) or less and therefore the FPF value of an aerosolised powder is a measure of the percentage of particles with the desired respirable size. A delivery device with a high FPF value is therefore desirable. Conventionally known DPI's provide an FPF of about 20 - 30 % w/w.

One measure of the efficiency of a delivery device is the difference between the metered dose (MD) and the delivered dose (DD), conventionally this is known as the retention. Thus, a delivery device with low retention is desirable. Conventionally known DPI's provide a powder retention of approximately 10 % w/w.

Conventionally known DPI's that provide a high FPF will provide a relatively high powder retention. Alternatively, those DPI's that provide a low powder retention may provide a relatively low FPF.

The delivery device of the invention is advantageous in that, *inter alia,* it provides a high FPF and a low powder retention. The achievement of a combined high FPF and low retention in a dry powder inhaler is novel *per se.*

Thus, according to a further aspect of the invention we provide a powder delivery device characterised in that the delivery device provides a high FPF and low powder retention.

In a preferred aspect of the invention the delivery device of the invention is a dry powder inhaler.

Thus we especially provide a delivery device as hereinbefore described which comprises a substantially axial jet and a substantially annular deagglomeration chamber.

The dry powder inhaler may therefore provide an FPF of at least 70% w/w. Preferably, the dry powder inhaler of the invention may provide an FPF of at least 80% w/w, more preferably at least 90% w/w and most preferably at least 95% w/w. The dry powder inhaler of the invention may provide a powder retention of less than 10% w/w, preferably less than 5% w/w and most preferably less than 2% w/w.

The invention will now be described by way of example only and with reference to the accompanying drawings in which:

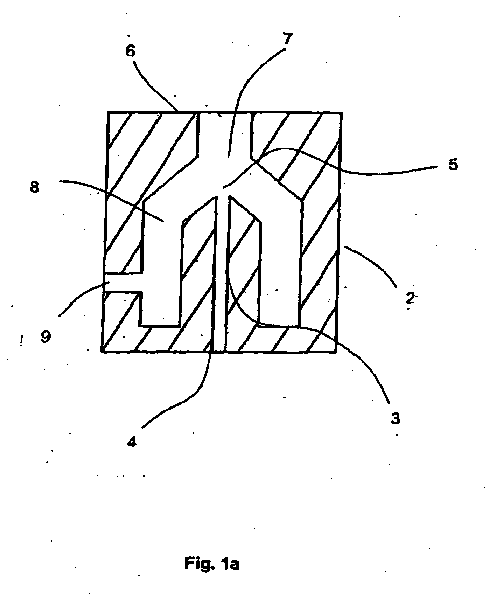

Figure 1a is a schematic cross-section of an air amplifying system of an embodiment of the invention;
Figure 1 is a perspective representation of a single chamber device of an embodiment of the invention;
Figure 2 is a perspective representation of a disassembled single chamber device of an embodiment of the invention;
Figure 3 is a cross-sectional representation of a single chamber device of an embodiment of the invention;
Figure 4 is a perspective representation of a multi axial nozzle device of an embodiment of the invention;
Figure 5 is a perspective representation of a disassembled multi axial nozzle device of an embodiment of the invention;
Figure 6 is a cross-sectional representation of a multi axial device of an embodiment of the invention;
Figure 7 is a perspective representation of a multi axial nozzle device of an embodiment of the invention provided with a plurality of powder inlets;
Figure 8 is a perspective representation of a disassembled multi axial nozzle device of an embodiment of the invention provided with a plurality of powder inlets;
Figure 9 is a cross-sectional view of a multi axial nozzle device of an embodiment of the invention provided with a plurality of powder inlets;
Figure 10 is a perspective representation of a multijet device of an embodiment of the invention;
Figure 11 is a cut-away perspective representation of a disassembled multijet device of an embodiment of the invention;
Figure 12 is a cross-sectional view of a multijet device of an embodiment of the invention;
Figure 13 is a perspective representation of a multijet device with individually activatable powder inlets;
Figure 14 is a cut-away perspective of a disassembled multijet device with individually activatable powder inlets; and
Figure 15 is a cross-sectional representation of the device of Figure 14.
Figures 16a-c are cross-sectional schematic representation of the system of an embodiment of the invention, illustrating the sequence of operation of the inhaler;
Figure 17 is a system of an embodiment of the invention;
Figure 18a is a mathematical model of a static pressure contour plot throughout the device of an embodiment of the invention;
Figure 18b is a mathematical model of a static pressure contour plot throughout a device of the prior art with main fluid flow from the side and powder flow through the middle;
Figure 19a is a mathematical model of a velocity magnitude contour plot throughout the device of an embodiment of the invention;
Figure 19b is a mathematical model of a velocity magnitude contour plot throughout a device of the prior art with main fluid flow from the side and powder flow through the middle;
Figure 20a is a mathematical model of the velocity of 5 µm particles with a coefficient of restitution of 0.25, throughout the device of an embodiment of the invention;
Figure 20b is a mathematical model of the velocity of 5 µm particles with a coefficient of restitution of 0.25, throughout a device of the prior art with main fluid flow from the side and powder flow through the middle;
Figure 21 a is a mathematical model of the velocity of 5 µm particles with a coefficient of restitution of 0.75, throughout the device of an embodiment of the invention;
Figure 21b is a mathematical model of the velocity of 5 µm particles with a coefficient of restitution of 0.75, throughout a device of the prior art with main fluid flow from the side and powder flow through the middle;
Figure 22a is a schematic representation of the device of an embodiment of the invention used in the experiment of Example 2; and
Figure 22b is a schematic representation of the device of the prior art, with main fluid flow from the side and powder flow through the middle, used in the experiment of Example 2.

Referring to Figure 1a, an air amplifying system (1) comprises a housing (2) and a fluid jet (3) provided with a fluid inlet (4) and a fluid outlet (5). The fluid outlet (5) is linked to an outlet nozzle (6) via an amplifying passage (7). The amplifying passage is also linked to an annular powder flow chamber (8). The powder flow chamber (8) is adapted to provide a non-laminar flow path for the powder (not shown). The chamber (8) is also provided with an inlet (9).

In use, fluid travelling through the amplifying jet (3) draws extraneous air through the powder chamber (8) aerosolising and deagglomerating the powder.

Referring to figures 1 to 3, a powder delivery device (111) comprises a body portion (112), a powder inlet (113) and powder outlet (114). The body portion (112) comprises a male member (115) and a female member (116). The male member (115) is a substantially cylindrical member provided with a frusto conical region (117) at one end (118). The second end (119) is provided with an annular shoulder (120), the shoulder (120) being provided with an annular recess (121) adapted to receive an annular sealing ring (122). The male member (115) is provided with an axial fluid flow chamber (123) provided with an inlet (124) and an outlet (125).

The female member (116) is adapted to fit over the male member (115). Thus the female member (116) presents a cavity which is provided with a frusto conical region (127) adapted to fit with the frusto conical region (117) of the male member (115). The region of the cavity distal to the frusto conical region (127) engages with the shoulder (120) of the male member (115). The end of the female member (116) is also provided with an annular recess (129) to receive a portion of the annular sealing ring (122). Thus, an annular powder dispersion chamber (130) is created between the male member (115) and the female member (116). The powder dispersion chamber (130) is provided with an inlet (131) and an outlet (132), the outlet (132) being coincident with the outlet (125) of the fluid flow chamber (123) and the outlet (114) of the female member (116).

In use, a gas, e.g. air or a volatile propellant passes along the fluid flow chamber (123), exiting at the outlet (125). At the junction with the powder dispersion chamber (130), the Venturi-type effect of the fluid flow creates a vacuum in the powder dispersion chamber (130) causing air to be drawn in at inlet (131).

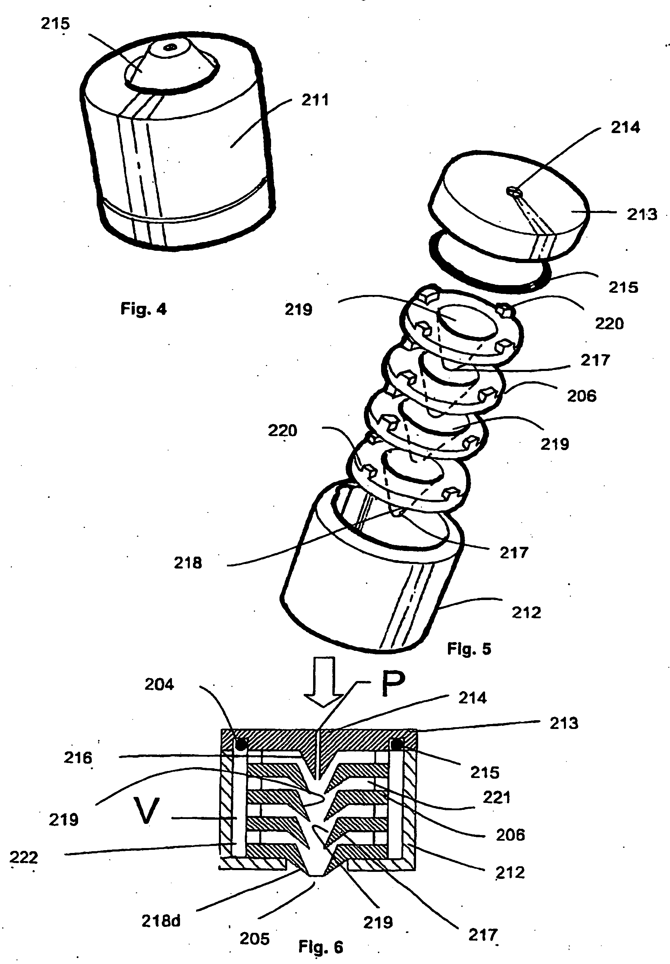

Referring to figures 4 to 6, a powder delivery device (211) comprises an annular body portion (212), and an end cap (213). The end cap (213) is provided with a central aperture (214) which is coincident with an aperture (205) created in the body portion (212). The end cap (213) is provided with an annular recess (204) adapted to receive an annular sealing ring (215). The end cap (213) is also provided with a frusto conical region (216) which surrounds the gas inlet aperture (214). The body portion (212) is also provided with a plurality of annular jet rings (206). Each jet ring (206) is provided with a hollow frusto conical portion (217) which surrounds a central jet aperture (218). Due to the hollow region (219) in the frusto conical portion (217), the portion acts as a female member which fits over a male member of the adjacent jet ring (206).

Each annular jet ring (206) is provided with a plurality of spacers (220) to separate one ring from an adjacent ring. The space (221) between each ring (206) can therefore act as a powder inlet for powder fed from cavity (222).

In use, a gas is fed through the inlet aperture (214) which passes through corresponding jet ring apertures (218), thus creating an increased vacuum in cavity (222). Powder is fed into cavity (222) and expressed through the jets and exits via aperture of the end jet (218d).

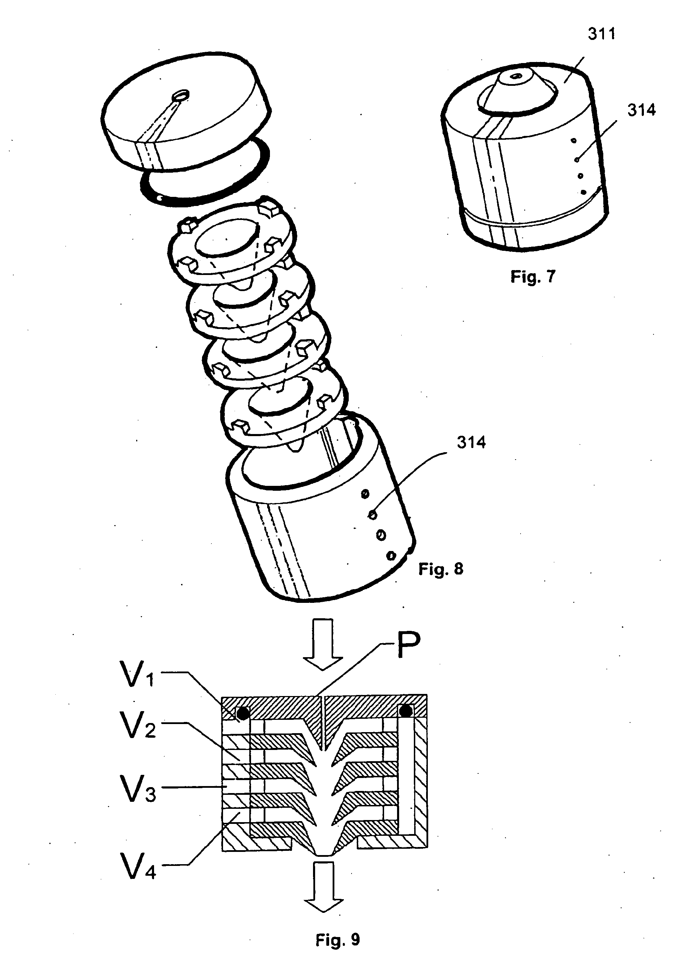

Referring to figures 7 to 9, a powder delivery device (311) is analogous to that illustrated in figures 4 to 6. The body of the device is provided with a plurality of apertures (314) which act as powder inlets.

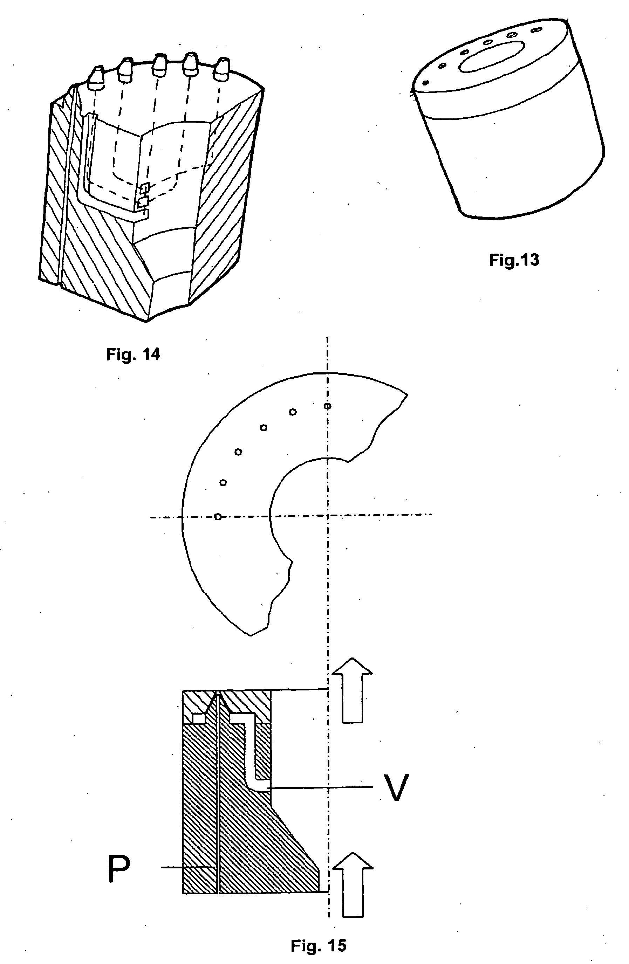

Referring to figures 10 to 15, a powder delivery device (411) comprises a body portion (412), a powder inlet (413) and a plurality of powder outlets (414). The body portion (412) comprises an annular wall (415) and end piece (416). The end piece (416) is provided with a gas inlet, an annular shoulder (417) and an annular recess (418) adapted to receive an annular sealing ring (419). The end piece (416) is provided with a male member region (427) which engages with a jet holder (420). The jet holder (420) is provided with a plurality of jets (421) and is provided with a recess region (422) and surface (423) facing the end piece (416). The recess (422) presents a cavity (403) which is beneath the jets (421). The jets (421) mate with powder outlets (414). Each of the jets comprises a frusto conical member (424) such that the shoulder of the cone (425) prevents the body portion (412) from resting against the jet holder (420) creating a powder delivery chamber (426).

In use, a gas, e.g. air or a volatile propellant passes through the inlet into cavity (403) and through jets (421). The Venturi-type effect of the fluid flow creates a vacuum in the powder dispersion chamber (426) causing powder to be drawn in at inlet (413).

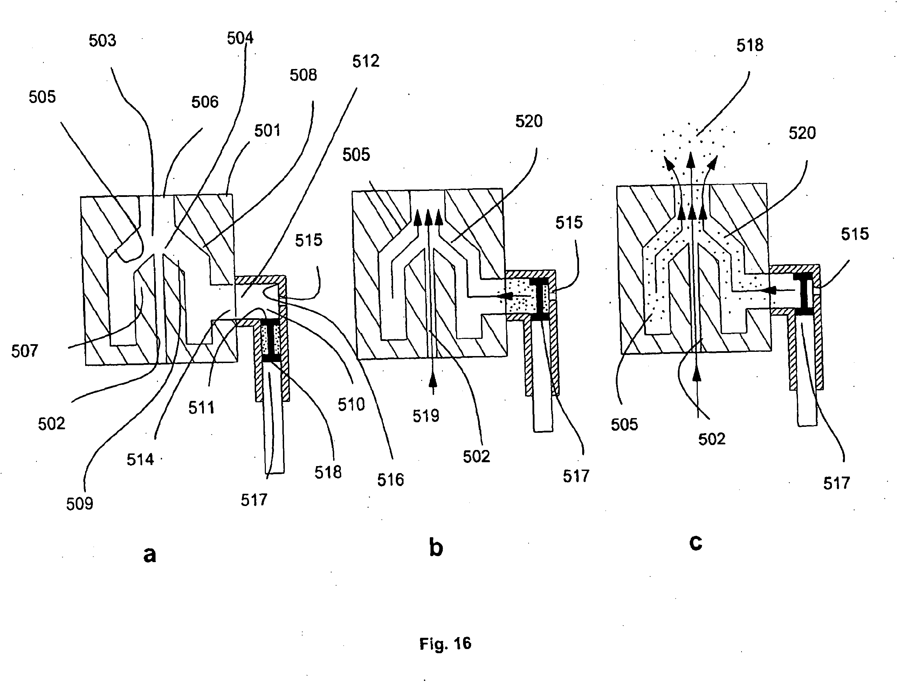

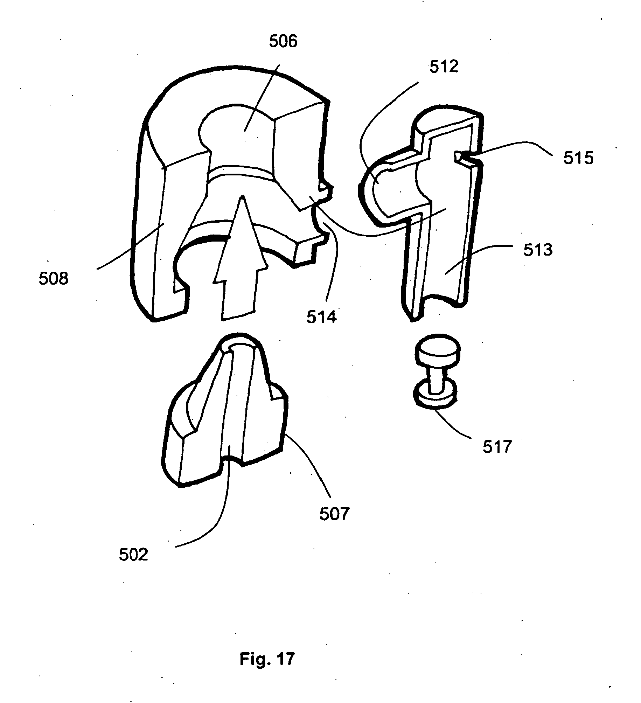

Referring to figures 16 and 17, a powder delivery system (501) comprises an axial fluid jet (502) with an outlet (503) at one end (504). The jet (502) is surrounded by an annular powder deagglomeration chamber (505). The outlet (503) of the jet (502) meets the deagglomeration chamber (505) and exits the system (501) at a nozzle (506). The deagglomeration chamber is comprised of a frusto conical male member (507) and a corresponding female member (508). The deagglomeration chamber (505) is provided, at one side with a powder delivery chamber (510).

The chamber (510) is provided with an inlet (511) and an outlet (512), at the end of an inlet conduit (513). The outlet (512) is coincident with an inlet (514) into the deagglomeration chamber (505). The delivery chamber (510) is also provided with an air inlet (515) positioned at the end (516) of the delivery chamber (510) distal to the outlet (512). In use, the inlet conduit (513) houses a metering spool (517) which carries a powdered medicament (518).

Referring to figures 16b and 16c, in use, the powder delivery system (501) is primed by inserting a spool (517) carrying powder (518) into the inlet conduit (513). The spool (517) is pushed to the end of the conduit (513) and into the delivery chamber (510). Compressed air is applied (see arrow (519)) into the jet (502). Air flows through the jet (502) and leaves at the outlet (503). The exiting air creates a vacuum in the deagglomeration chamber (505) and causes suction in the powder delivery chamber (510).

The air flow (shown by arrow 520) deagglomerates and subsequently aerosolises the powder (518) which passes out of the delivery chamber (510). Fine particles are capable of passing straight through the deagglomeration chamber (505) whilst larger particles collide with the walls of the chamber (505) (and possibly with each other)) and are deagglomerated to small respirable particles when they reach the nozzle (506).

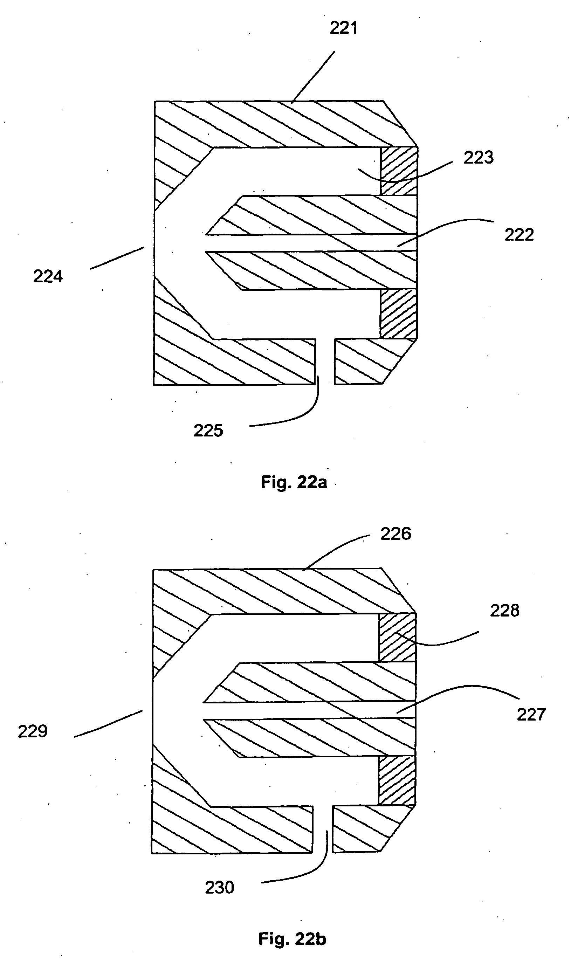

Referring to Figure 22; in Figure 22a (Case 1) an air amplifier of the invention (221), comprises a central, axial jet (222), an annular powder deagglomeration chamber (223) and a nozzle (224)), the annular deagglomeration chamber being provided with a powder inlet (225).

In Figure 22b (Case 2) an air amplifier of the prior art (226), comprises a central, axial powder chamber (227), an annular jet chamber (228) and a nozzle (229)), the annular jet chamber (227) being provided with an air inlet (230).

### Example 1

### CFD Examination of the flow

### Objectives of study:

To generate a CFD model of the flow of air through two configurations of the air amplifier of the invention.

To validate the air flow model by comparison with experimental data relating to prototype air amplifiers.

### Geometry and grid

A mesh was produced in ICEM-CFD using 235590 cells. Tetrahedral cells were used in the converging cone section of the geometry. Pentahedral cells were used in the straight pipe sections to reduce cell numbers over a fully tetrahedral mesh.

### Model inputs - continuous phase

A constant absolute pressure equal to atmospheric was set at the outflow boundaries (i.e., a gauge pressure of 0Pa)

An ideal gas law was used for the fluid properties to allow for the compressibility of air at the high Mach numbers induced.

The segregated solver was used, along with second-order upwind discretisation for all variables, and the SIMPLE velocity-pressure coupling algorithm.

Standard k-ε turbulence model was used with viscous heating.

### Boundary conditions:

### Case 1: Flow through centre, drug from side

| *Region* | *Value* | *Turbulent Intensity* | *Length scale* |
|---|---|---|---|
| *Middle inlet* | 3Bar | 5% | 0.0001 m |
| *Side inlet* | 1.735e-5kg/s | 5% | 0.0001 m |
| *Outlet* | 0 Pa | 1% | 0.00005 m |

### Case 2: Flow from side, drug through middle

| *Region* | *Value* | *Turbulent Intensity* | *Length scale* |
|---|---|---|---|
| *Middle inlet* | 1.735e-5kg/s | 5% | 0.0001 m |
| *Side inlet* | 3Bar | 5% | 0.0001 m |
| *Outlet* | 0 Pa | 1% | 0.00005 m |

### Model inputs - discrete phase

Density of drug particles was set at 800 kg m⁻³.

The total mass flow rate was set to 1.12×10⁻⁵ kg/s (equal to 2.8mg in .25 seconds, value supplied by IB).

The continuous phase and discrete phases were simulated both as coupled and uncoupled.

The discrete phase boundary condition at wall surfaces was set to "reflect" with a coefficient of restitution of 0.25 and 0.75.

Turbulent interaction between the continuous and discrete phase was modelled.

Initial particle speed was set to the average velocity magnitude over the inflow boundary from which the particles were released.

Initial particle of 293K.

Particle size simulated 5 microns.

The drag law used for the simulations was the high-Mach-number drag law to account for the high velocities and large variations in fluid density seen in the simulations.

Number of particles tracked was 60 spaced evenly over the drug inlet boundary.

### Results and Conclusions

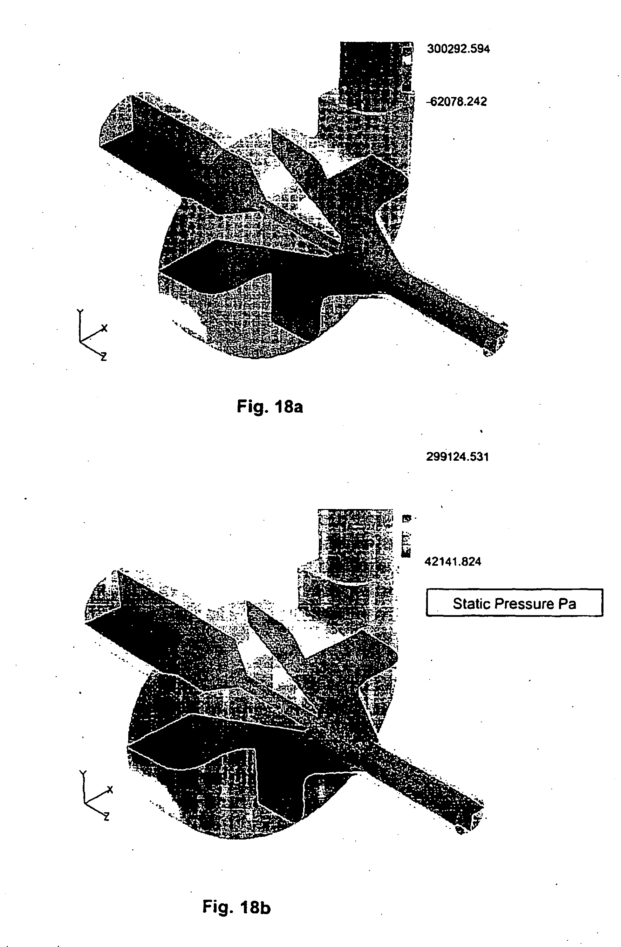

Figures 18a and 18b show a static pressure contour plot from Case 1 and Case 2 respectively.

A preliminary CFD study of the section pressure with the side inlet blocked of to flow showed a negative static pressure of 529mbar. Experimental results taken at IB give a suction pressure of 334mbar on the side inlet with a driving pressure of 3bar on the central inlet. This corresponds sufficiently well to the CFD prediction to proceed with the study of particle trajectories. In Case 1 the flow through the centre induces a suction pressure of 122mbar on the side inlet. Thus, as expected, the presence of the flow through the side inlet changes the pressure distribution.

Both the CFD and experimental results show that a positive 3 bar pressure on the side inlet does not induce a suction pressure on the central inlet. Thus, in Case 2 a positive pressure on the central inlet is needed to induce the correct mass flow rate (to allow comparison between the two cases).

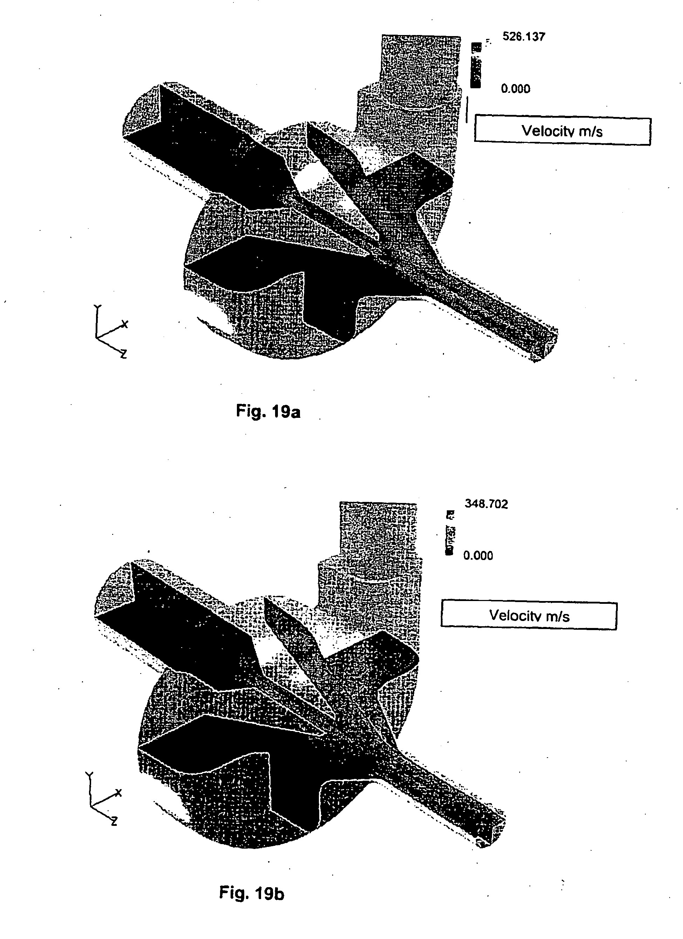

Figures 19a and 19b show a velocity magnitude contour plot from Case 1 and Case 2 respectively.

As expected, in Case 1 the highest velocity (>500m/s) occurs when the flow expands as it leaves the smallest diameter pipe through the middle of the device and corresponds to the region where the air density is at its lowest.

In Case 2 the highest velocity seen is smaller (>300m/s) and is in the outlet section of the device. There is no point where the flow is accelerated sufficiently for the density of the air to decrease below its value at standard temperature and pressure, unlike Case 1. It is the expansion of air in Case 1 that drives the larger velocities.

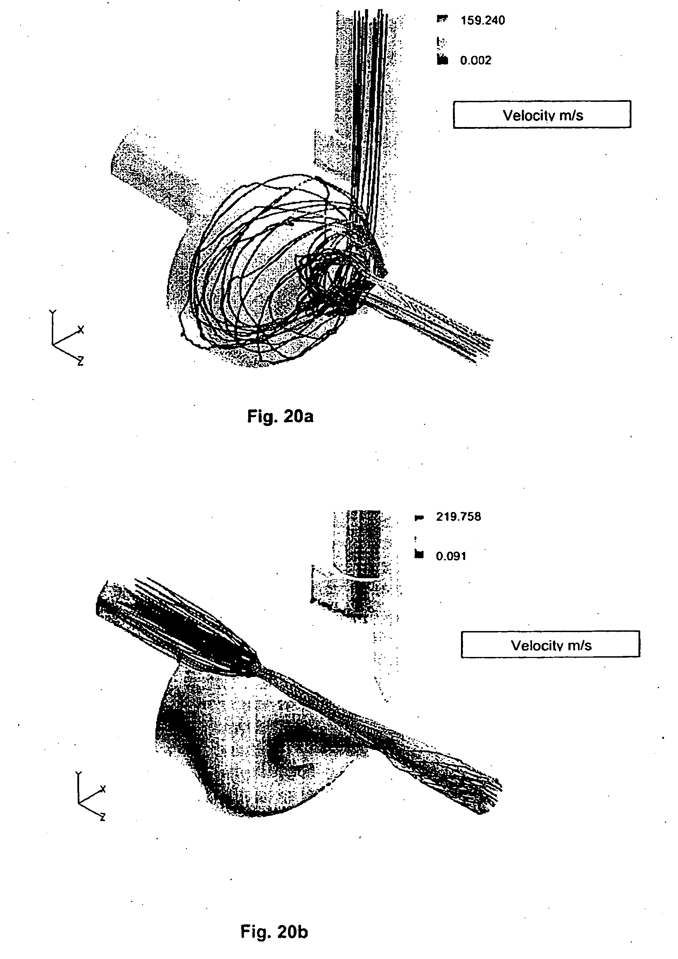

Figures 20a and 20b show the trajectories of 5 micron particles from Case 1 and Case 2 respectively when a coefficient of restitution of 0.25 is used. 5 micron particles have small enough inertia that they are strongly effected by local air speeds.

In Case 1 particles are thrown out to the outside of the converging vortex section and are then entrained into the boundary layers of the exit flow. Turbulent eddies can knock the trajectories nearer into the middle of the flow but they tend to follow the outside of the exiting flow.

In Case 2 the small inertia of the particles mean that they largely follow the flow and do not collide with the wall surfaces. The particle trajectory simulations suggest that wall collisions are limited to the converging section of the "(central) drug inlet" and the outlet section.

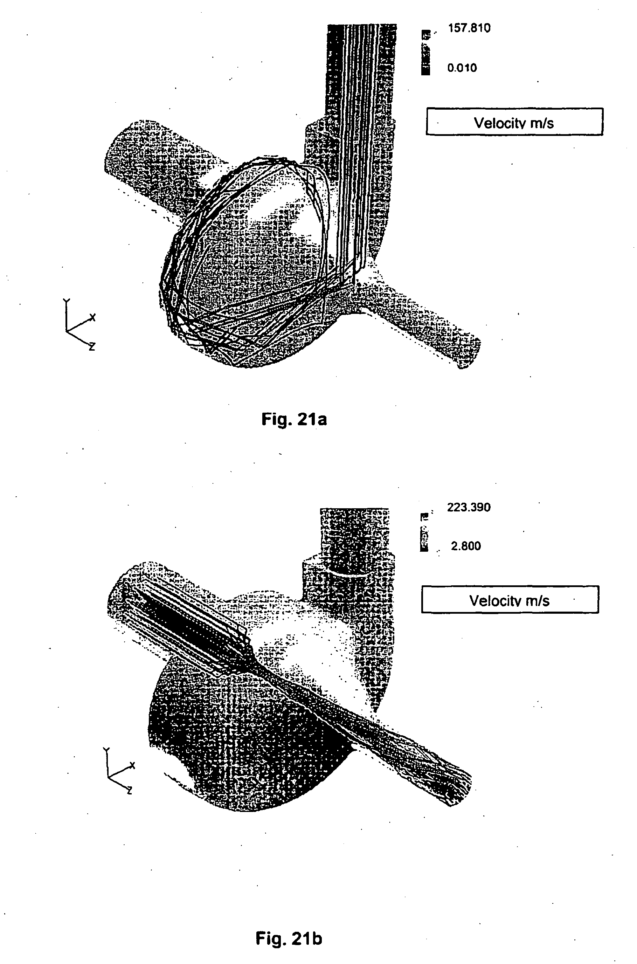

Figures 21a and 21b show the trajectories of 5 micron particles from Case 1 and Case 2 respectively when a coefficient of restitution of 0.75 is used. The coefficient change has little effect on the overall results from Case 2 because the particles do not often collide with the walls. In Case 1 the coefficient change results in less momentum being lost at each collision in the converging annulus section. Thus the particles have enough momentum to remain in the rotating flow for longer. They are drawn down into the converging section where eventually they are entrained into the main flow and through the outlet.

N.B. The image shows only 2 of the 12 particles tracked for the image exiting the outlet. This is because the files produced when the particles remain bouncing around the domain, are very large, therefore they are truncated for speed.

### Example 2

### FPF and FPD Measurements

### Method

The objectives of the test were to assess and record the performance of two main variants of the air amplifier system based on evaluation of the fine particle fraction (FPF) and the powder pick-up or delivered dose (DD) and to access any blockage characteristics of the systems, if any. The test work required the use of a steel amplifier system according to figure 22 manufactured at IB Tewkesbury to enable precise and controlled setting of the geometries and orientations of the system. In addition plastic injection moulded components of the variants were manufactured from the same facility and tested in the similar using a similar protocol. Air amplifier variants were tested using a spray dried leucine preparation to represent the powder characteristics of a typical formulation for systemic drug delivery via the lung. FPF and DD were determined gravimetrically using a modified glass twin impinger apparatus. Each variant was tested using a two second pulse of air with feed pressures of 5.7 and 3 bar. The two key amplifier system variants were those which delivered powder through the "non linear, conical" side passage (the preferred embodiment denoted A (or Case 1 in the CFD study)) and a variant B (or Case 2 in the CFD study) which delivered powder through the "linear central passage". The results are illustrated in Table 1.

**Table 1**

| **Variant** | **Offset (mm)** | **Jet Dia. (µm)** | **Nozzle Dia. (µm)** | **Feed Press. (bar)** | **Average MD (mg)** | **Average DD(mg)** | **FPD (mg)** | **FPF (%)** |
|---|---|---|---|---|---|---|---|---|
| A | 1.0 | 250 | 500 | 5.7 | 1.04 | 1.04 | 0.74 | 71.2% |
| A | 1.0 | 250 | 500 | 5.7 | 1.16 | 1.16 | 0.82 | 70.7% |
| B₅* | 0.1 | 500 | 500 | 3.0 | - | - | - | - |
| B₅* | 0.1 | 500 | 500 | 5.5 | - | - | - | - |
| B₆₅ | 0.1 | 500 | 650 | 3.0 | 1.04 | 1.04 | 0.38 | 36.5% |
| B₆₅ | 0.1 | 500 | 650 | 5.5 | 1.12 | 1.12 | 0.39 | 34.8% |
| B₇₅ | 0.1 | 500 | 750 | 3.0 | 1.1 | 1.1 | 0.37 | 33.6% |
| B₇₅ | 0.1 | 500 | 750 | 5.5 | 1.14 | 1.14 | 0.43 | 37.7% |
| B₈₅ | 0.1 | 500 | 850 | 3.0 | 1.22 | 1.22 | 0.48 | 393% |
| B₈₅ | 0.1 | 500 | 850 | 5.5 | 0.92 | 0.92 | 0.37 | 40.2% |

| | | | | | | | | |
|---|---|---|---|---|---|---|---|---|
| * No FPD or FPF - nozzle blocked | | | | | | | | |

## Claims

1. An air amplifying system comprising an amplifying fluid jet (3; 123; 502) provided with a fluid inlet (4; 124) and a fluid outlet (5; 125; 503), the fluid outlet (5; 125; 503) being linked to an outlet nozzle (6; 114; 506) via an amplifying passage (7), the amplifying passage (7) also being linked to a powder chamber (8; 130; 505), said chamber being adapted for non-laminar powder flow, such that fluid travelling from the fluid outlet (5; 125; 503) of the jet draws extraneous air and aerosolised powder through the powder chamber (8; 130; 505) so that the extraneous air and aerosolised powder mix with the amplifying fluid in the amplifying passage (7) and the amplified mixture exits through the outlet nozzle (6; 114; 506) **characterised in that** the powder chamber (8; 130; 505) is an annular chamber which is adapted to provide non-laminar flow of the powder.

2. An air amplifying system according to claim 1 **characterised in that** the vacuum created by the exit of the gas stream from the amplifying jet (3; 123; 502) creates a vacuum in the powder chamber (8; 130; 505) and an entrainment air flow through a powder.

3. An air amplifying system according to claim 2 **characterised in that** the entrainment air flow is sufficient to cause deagglomeration and/or entrainment and then subsequent aerosolisation of the powder.

4. An air amplifying system according to claim 1 **characterised in that** the system comprises an annular powder chamber (8; 130; 505) and an axial fluid jet (3; 123; 502).

5. An air amplifying system according to claim 4 **characterised in that** the powder chamber (8; 130; 505) is substantially circumferential to the body of the amplifying system and the fluid jet (3; 123; 502) is axial to the body.

6. An air amplifying system according to claim 1 **characterised in that** the annular powder chamber (130) comprises a male (115) and a female portion (116).

7. An air amplifying system according to claim 6 **characterised in that** the outlet end (125) of the fluid jet (123) comprises a frustoconical male member (115) which fits into a female portion (116) of the powder chamber (130).

8. An air amplifying system according to claim 6 **characterised in that** the separation between the male (115) and female (116) members (the clearance) is from 500 to 2000 µm.

9. An air amplifying system according to claim 8 **characterised in that** the clearance is about 1000 µm.

10. An air amplifying system according to claim 9 **characterised in that** the diameter of the jet (123) is from 100 to 500 µm.

11. An air amplifying system according to claim 10 **characterised in that** diameter of the jet (123) is from 200 to 300 µm.

12. An air amplifying system according to claim 11 **characterised in that** diameter of the jet (123) is about 250 µm.

13. An air amplifying system according to claim 1 **characterised in that** the diameter of the nozzle (6; 114; 506) is from 100 µm to 1500 µm.

14. An air amplifying system according to claim 13 **characterised in that** the diameter of the nozzle (6; 114; 506) is from 400 µm to 1200 µm.

15. An air amplifying system according to claim 14 **characterised in that** the diameter of the nozzle (6; 114; 506) is from 400 µm to 600 µm.

16. An air amplifying system according to claim 15 **characterised in that** the diameter of the nozzle (6; 114; 506) is about 500 µm.

17. An air amplifying system according to claim 1 **characterised in that** the diameter of the nozzle (6; 114; 506) is greater than the diameter of the jet (3; 123; 502).

18. An air amplifying system according to claim 17 **characterised in that** the ratio of the diameter of the jet (3; 123; 502) to the diameter of the nozzle (6; 114; 506) is from 1:8 to 1:2.

19. An air amplifying system according to claim 18 **characterised in that** the ratio of the diameter of the jet (3; 123; 502) to the diameter of the nozzle (6; 114; 506) is from 1:4 to 1:2

20. An air amplifying system according to claim 19 **characterised in that** the ratio of the diameter of the jet (3; 123; 502) to the diameter of the nozzle (6; 114; 506) is 1:2.

21. An air amplifying system according to claim 1 **characterised in that** the chamber (505) is provided with a powder reservoir or a powder metering member adjacent an inlet (514) to the powder chamber.

22. An air amplifying system according to claim 21 **characterised in that** the powder reservoir and/or metering member is contiguous with the powder chamber (505).

23. An air amplifying system according to claim 21 **characterised in that** the powder chamber (505) is connected to the powder reservoir and/or metering member by one or more conduits (513).

24. An air amplifying system according to claim 1 **characterised in that** the system comprises a plurality of nozzles (414).

25. An air amplifying system according to claim 1 **characterised in that** the system comprises a plurality of fluid jets (421).

26. An air amplifying system according to claim 25 **characterised in that** the plurality of jets (421) comprises a plurality of interlocking jets.

27. An air amplifying system according to claim 26 **characterised in that** each jet (421) is provided with a powder inlet (413).

28. A powder delivery device which comprises a delivery passage, a powder reservoir and/or a metering member adapted to present a measured dose of powder to the delivery passage **characterised in that** the powder delivery device is provided with an air amplifying system according to claim 1.

29. A powder delivery device according to claim 28 **characterised in that** the air amplifying system creates an entrained air flow through the powder reservoir and/or metering member.

30. A powder delivery device according to claim 29 **characterised in that** the entrained air flows through the powder which is presented either direct from the reservoir or from the metering member.

31. A powder delivery device according to claim 30 **characterised in that** the entrained air inlet is positioned adjacent to a first side of the reservoir and/or metering member and a vacuum is created adjacent a second, opposite side of the reservoir and/or metering member.

32. A powder delivery device according to claim 29 **characterised in that** a further inlet tube is provided which is adapted to introduce entrainment air into the reservoir/metering member.

33. A powder delivery device according to claim 29 **characterised in that** the entrained air flow is sufficient to both deagglomerate and aerosolise the powder.

34. A powder delivery device according to claim 28 **characterised in that** the device is an inhalation device.

35. A powder delivery device according to claim 34 **characterised in that** the inhalation device is a dry powder inhaler.

36. A powder delivery device according to claim 34 **characterised in that** the amplifying system is arranged to provide the separate, sequential or simultaneous operation of the jet(s) to enable the creation of an aerosolised powder which coincides with the inspiration of a patient.

37. A powder delivery device according to claim 28 **characterised in that** the fluid used in the fluid jet is a gas.

38. A powder delivery device according to claim 37 **characterised in that** the gas is generated from the volatilisation of a volatile propellant.

39. A powder delivery device according to claim 28 **characterised in that** the fluid flow is generated by an electric motor.

40. A powder delivery device according to claim 28 **characterised in that** the fluid flow is generated by a manually primed piston.

41. A powder delivery device according to claim 38 **characterised in that** the propellant is a non-CFC propellant.

42. A powder delivery device according to claim 41 **characterised in that** the propellant is a hydrofluoroalkane (HFA).

43. A powder delivery device according to claim 42 **characterised in that** the propellant is a fluoroalkane.

44. A powder delivery device according to claim 43 **characterised in that** the fluoroalkane is a fluoromethane, a fluoroethane or a mixture of fluoroalkanes.

45. A powder delivery device according to claim 44 **characterised in that** the fluoroalkane is selected from the group trichlorofluoromethane, dichlorodifluoromethane, 1,2-dichlorotetrafluorethane, trichlorotrifluoroethane and chloropentafluoroethane.

46. A powder delivery device according to claim 45 **characterised in that** the fluoroalkane is HFA 134 (1,1,1,2-tetrafluoroethane).

47. A powder delivery device according to claim 46 **characterised in that** the fluoroalkane is HFA 227.

48. A powder delivery device according to claim 28 **characterised in that** the metering member comprises a powder housed in a spool and spool carrier.

49. A powder delivery device according to claim 28 **characterised in that** the metering member is a capsule.

50. A powder delivery device according to claims 48 or 49 **characterised in that** the device is provided with means for opening the metering member.

51. A powder delivery device according to claim 48 **characterised in that** the spool carrier is an integral part of the delivery device.

52. A powder delivery device according to claim 28 **characterised in that** the powder is selected from the group of drugs for the treatment of asthma, COPD or respiratory infections such as β₂-agonists; fenoterol, formoterol, pirbuterol, reproterol, rimiterol, salbutamol, salmeterol and terbutaline; non-selective beta-stimulants such as isoprenaline; xanthine bronchodilators; theophylline, aminophylline and choline theophyllinate; anticholinergics such as ipratropium bromide; mast cell stabilisers, such as sodium cromoglycate and ketotifen; bronchial anti-inflammatory agents, such as nedocromil sodium; steroids, such as beclomethasone dipropionate, fluticasone, budesonide and flunisolide; and combinations thereof.

53. A powder delivery device according to.claim 52 **characterised in that** the combination of powders is selected from steroids, such as beclomethasone dipropionate; fluticasone, budesonide and flunisolide; and combinations of (β₂-agonists, such as, formoterol and salmeterol.

54. A powder delivery device according to claim 28 **characterised in that** the powder is systemically active.

55. A powder delivery device according to claim 54 **characterised in that** the powder is selected from a proteinaceous compounds and/or macromolecules, such as hormones and mediators, such as insulin, human growth hormone, leuprolide and alpha interferon; growth factors, anticoagulants, immunomodulators, cytokines and nucleic acids.

56. A powder delivery device according to claim 28 **characterised in that** the powder is a combination selected from a powder as defined by claim 52 and a powder as defined by claim 53.

## Patentansprüche

1. Luftverstärkungssystem, umfassend ein Fluidverstärkungsgebläse (3; 123; 502), versehen mit einem Fluideinlass (4; 124) und einem Fluidauslass (5; 125; 503), wobei der Fluidauslass (5; 125; 503) über einen Verstärkungsdurchgang (7) mit einer Auslassdüse (6; 114; 506) verbunden ist, der Verstärkungsdurchgang (7) ebenfalls mit einer Pulverkammer (8; 130; 505) verbunden ist, die Kammer geeignet ist für nicht-laminaren Pulverfluss, dergestalt dass Fluid, welches aus dem Fluidauslass (5; 125; 503) des Gebläses austritt, Außenluft und in Aerosolform vernebeltes Pulver durch die Pulverkammer (8; 130; 505) so hindurch zieht, dass sich die Außenluft und das in Aerosolform vernebelte Pulvergemisch im Verstärkungsdurchgang (7) mit dem Verstärkungsfluid vermischen und das verstärkte Gemisch durch die Auslassdüse (6; 114; 506) hindurch austritt, **dadurch gekennzeichnet, dass** die Pulverkammer (8; 130; 505) eine ringförmige Kammer ist, welche dafür geeignet ist, für einen nicht-laminaren Fluss des Pulvers zu sorgen.

2. Luftverstärkungssystem nach Anspruch 1, **dadurch gekennzeichnet, dass** das durch das Austreten des Gasstroms aus dem Verstärkungsgebläse (3; 123; 502) erzeugte Vakuum ein Vakuum in der Pulverkammer (8; 130; 505) und einen Mitreiß-Luftfluss durch ein Pulver hindurch erzeugt.

3. Luftverstärkungssystem nach Anspruch 2, **dadurch gekennzeichnet, dass** der Mitreiß-Luftfluss ausreicht, um Entagglomeration und/oder Mitreißen und eine sich dann anschließende Vernebelung in Aerosolform des Pulvers zu bewirken.

4. Luftverstärkungssystem nach Anspruch 1, **dadurch gekennzeichnet, dass** das System eine ringförmige Pulverkammer (8; 130; 505) und ein axiales Fluidgebläse (3; 123; 502) umfasst.

5. Luftverstärkungssystem nach Anspruch 4, **dadurch gekennzeichnet, dass** die Pulverkammer (8; 130; 505) im Wesentlichen umfänglich zum Körper des Verstärkungssystems ist und das Fluidgebläse (3; 123; 503) axial zum Körper ist.

6. Luftverstärkungssystem nach Anspruch 1, **dadurch gekennzeichnet, dass** die ringförmige Pulverkammer (130) einen Außenabschnitt (115) und einen Innenabschnitt (116) umfasst.

7. Luftverstärkungssystem nach Anspruch 6, **dadurch gekennzeichnet, dass** das Auslassende (125) des Fluidgebläses (123) ein kegelstumpfförmiges Außenelement (115) umfasst, welches in einen Innenabschnitt (116) der Pulverkammer (130) hineinpasst.

8. Luftverstärkungssystem nach Anspruch 6, **dadurch gekennzeichnet, dass** der Abstand zwischen dem Außenelement (115) und dem Innenelement (116) (der Zwischenraum) im Bereich von 500 bis 2000 µm liegt.

9. Luftverstärkungssystem nach Anspruch 8, **dadurch gekennzeichnet, dass** der Zwischenraum zirka 1000 µm beträgt.

10. Luftverstärkungssystem nach Anspruch 9, **dadurch gekennzeichnet, dass** der Durchmesser des Gebläses (123) im Bereich von 100 bis 500 µm liegt.

11. Luftverstärkungssystem nach Anspruch 10, **dadurch gekennzeichnet, dass** der Durchmesser des Gebläses (123) im Bereich von 200 bis 300 µm liegt.

12. Luftverstärkungssystem nach Anspruch 11, **dadurch gekennzeichnet, dass** der Durchmesser des Gebläses (123) zirka 250 µm beträgt.

13. Luftverstärkungssystem nach Anspruch 1, **dadurch gekennzeichnet, dass** der Durchmesser der Düse (6; 114; 506) im Bereich von 100 µm bis 1500 µm liegt.

14. Luftverstärkungssystem nach Anspruch 13, **dadurch gekennzeichnet, dass** der Durchmesser der Düse (6; 114; 506) im Bereich von 400 µm bis 1200 µm liegt.

15. Luftverstärkungssystem nach Anspruch 14, **dadurch gekennzeichnet, dass** der Durchmesser der Düse (6; 114; 506) im Bereich von 400 µm bis 600 µm liegt.

16. Luftverstärkungssystem nach Anspruch 15, **dadurch gekennzeichnet, dass** der Durchmesser der Düse (6; 114; 506) zirka 500 µm beträgt.

17. Luftverstärkungssystem nach Anspruch 1, **dadurch gekennzeichnet, dass** der Durchmesser der Düse (6; 114; 506) größer ist als der Durchmesser des Gebläses (3; 123; 502).

18. Luftverstärkungssystem nach Anspruch 17, **dadurch gekennzeichnet, dass** das Verhältnis des Durchmessers des Gebläses (3; 123; 502) zum Durchmesser der Düse (6; 114; 506) im Bereich von 1:8 bis 1:2 liegt.

19. Luftverstärkungssystem nach Anspruch 18, **dadurch gekennzeichnet, dass** das Verhältnis des Durchmessers des Gebläses (3; 123; 502) zum Durchmesser der Düse (6; 114; 506) im Bereich von 1:4 bis 1:2 liegt.

20. Luftverstärkungssystem nach Anspruch 19, **dadurch gekennzeichnet, dass** das Verhältnis des Durchmessers des Gebläses (3; 123; 502) zum Durchmesser der Düse (6; 114; 506) 1:2 ist.

21. Luftverstärkungssystem nach Anspruch 1, **dadurch gekennzeichnet, dass** die Kammer (505) ein Pulverbehältnis oder ein Pulverdosierelement angrenzend an einen Einlass (514) zur Pulverkammer aufweist.

22. Luftverstärkungssystem nach Anspruch 21, **dadurch gekennzeichnet, dass** das Pulverbehältnis und/oder das Dosierelement an die Pulverkammer (505) angrenzen.

23. Luftverstärkungssystem nach Anspruch 21, **dadurch gekennzeichnet, dass** die Pulverkammer (505) mit dem Pulverbehältnis und/oder dem Dosierelement durch einen oder mehr Kanäle (513) verbunden ist.

24. Luftverstärkungssystem nach Anspruch 1, **dadurch gekennzeichnet, dass** das System eine Vielzahl von Düsen (414) umfasst.

25. Luftverstärkungssystem nach Anspruch 1, **dadurch gekennzeichnet, dass** das System eine Vielzahl von Fluidgebläsen (421) umfasst.

26. Luftverstärkungssystem nach Anspruch 25, **dadurch gekennzeichnet, dass** die Vielzahl von Gebläsen (421) eine Vielzahl von ineinandergreifenden Gebläsen umfasst.

27. Luftverstärkungssystem nach Anspruch 26, **dadurch gekennzeichnet, dass** jedes Gebläse (421) einen Pulvereinlass (413) aufweist.

28. Pulverzuführungsvorrichtung, welche einen Zuführungsdurchgang, ein Pulverbehältnis und/oder ein Dosierelement umfasst, das dafür geeignet ist, dem Zuführungsdurchgang eine abgemessene Dosis Pulver zu präsentieren, **dadurch gekennzeichnet, dass** die Pulverzuführungsvorrichtung ein Luftverstärkungssystem nach Anspruch 1 aufweist.

29. Pulverzuführungsvorrichtung nach Anspruch 28, **dadurch gekennzeichnet, dass** das Luftverstärkungssystem einen mitgerissenen Luftfluss durch das Pulverbehältnis und/oder durch das Dosierelement hindurch erzeugt.

30. Pulverzuführungsvorrichtung nach Anspruch 29, **dadurch gekennzeichnet, dass** die mitgerissene Luft durch das Pulver hindurch fließt, welches entweder direkt vom Behältnis aus oder vom Dosierelement aus präsentiert wird.

31. Pulverzuführungsvorrichtung nach Anspruch 30, **dadurch gekennzeichnet, dass** der Einlass für die mitgerissene Luft benachbart zu einer ersten Seite des Behältnisses und/oder des Dosierelements positioniert ist, und dass ein Vakuum benachbart zu einer zweiten, gegenüberliegenden Seite des Behältnisses und/oder des Dosierelements geschaffen wird.

32. Pulverzuführungsvorrichtung nach Anspruch 29, **dadurch gekennzeichnet, dass** ein weiterer Einlassschlauch bereitgestellt ist, der dafür geeignet ist, Mitreißluft in das Behältnis/in das Dosierelement einzubringen.

33. Pulverzuführungsvorrichtung nach Anspruch 29, **dadurch gekennzeichnet, dass** der mitgerissene Luftfluss ausreicht, um das Pulver sowohl zu entagglomerisieren als auch in Aerosolform zu vernebeln.

34. Pulverzuführungsvorrichtung nach Anspruch 28, **dadurch gekennzeichnet, dass** die Vorrichtung eine Inhalationsvorrichtung ist.

35. Pulverzuführungsvorrichtung nach Anspruch 34, **dadurch gekennzeichnet, dass** die Inhalationsvorrichtung ein Trockenpulverinhalator ist.

36. Pulverzuführungsvorrichtung nach Anspruch 34, **dadurch gekennzeichnet, dass** das Verstärkungssystem angeordnet ist, um für das separate, sequentielle oder gleichzeitige Betreiben des Gebläses/der Gebläse zu sorgen, um die Erzeugung eines in Aerosolform vernebelten Pulvers zu ermöglichen, das mit der Einatmung eines Patienten zusammenfällt.

37. Pulverzuführungsvorrichtung nach Anspruch 28, **dadurch gekennzeichnet, dass** das im Fluidgebläse verwendete Fluid ein Gas ist.

38. Pulverzuführungsvorrichtung nach Anspruch 37, **dadurch gekennzeichnet, dass** das Gas aus der Verflüchtigung eines flüchtigen Treibmittels erzeugt wird.

39. Pulverzuführungsvorrichtung nach Anspruch 28, **dadurch gekennzeichnet, dass** der Fluidfluss durch einen Elektromotor erzeugt wird.

40. Pulverzuführungsvorrichtung nach Anspruch 28, **dadurch gekennzeichnet, dass** der Fluidfluss durch einen manuell zum Ansaugen gebrachten Kolben erzeugt wird.

41. Pulverzuführungsvorrichtung nach Anspruch 38, **dadurch gekennzeichnet, dass** das Treibmittel ein Nicht-CFC-Treibmittel ist.

42. Pulverzuführungsvorrichtung nach Anspruch 41, **dadurch gekennzeichnet, dass** das Treibmittel ein Fluorwasserstoffalkan (HFA) ist.

43. Pulverzuführungsvorrichtung nach Anspruch 42, **dadurch gekennzeichnet, dass** das Treibmittel ein Fluoralkan ist.

44. Pulverzuführungsvorrichtung nach Anspruch 43, **dadurch gekennzeichnet, dass** das Fluoralkan ein Fluormethan, ein Fluorethan oder ein Gemisch von Fluoralkanen ist.

45. Pulverzuführungsvorrichtung nach Anspruch 44, **dadurch gekennzeichnet, dass** Fluoralkan ausgewählt wird aus der Gruppe Trichlorfluormethan, Dichlordifluormethan, 1,2-Dichlor-tetrafluorethan, Trichlortrifluorethan und Chlorpentafluorethan.

46. Pulverzuführungsvorrichtung nach Anspruch 45, **dadurch gekennzeichnet, dass** das Fluoralkan HFA 134 (1,1,1,2-Tetrafluor-ethan) ist.

47. Pulverzuführungsvorrichtung nach Anspruch 46, **dadurch gekennzeichnet, dass** das Fluoralkan HFA 227 ist.

48. Pulverzuführungsvorrichtung nach Anspruch 28, **dadurch gekennzeichnet, dass** das Dosierelement ein Pulver umfasst, welches in einer Spule und in einem Spulenträger untergebracht ist.

49. Pulverzuführungsvorrichtung nach Anspruch 28, **dadurch gekennzeichnet, dass** das Dosierelement eine Kapsel ist.

50. Pulverzuführungsvorrichtung nach Anspruch 48 oder 49, **dadurch gekennzeichnet, dass** die Vorrichtung ein Mittel für das Öffnen des Dosierelements aufweist.

51. Pulverzuführungsvorrichtung nach Anspruch 48, **dadurch gekennzeichnet, dass** der Spulenträger ein fester Bestandteil der Zuführungsvorrichtung ist.

52. Pulverzuführungsvorrichtung nach Anspruch 28, **dadurch gekennzeichnet, dass** das Pulver ausgewählt wird aus der Gruppe von Medikamenten für die Behandlung von Asthma, chronisch obstruktiver Lungenerkrankung (COPD) oder von Infektionen der Atemwegsorgane, wie zum Beispiel β₂-Agonisten; Fenoterol, Formoterol, Pirbuterol, Reproterol, Rimiterol, Salbutamol, Salmeterol und Terbutalin; nicht-selektive Beta-Stimulanzien, wie zum Beispiel Isoprenalin, Xanthin-Bronchodilatatoren, Theophyllin, Aminophyllin und Cholintheophyllinat; Anticholinergika, wie zum Beispiel lpratropiumbromid, Mastzellenstabilisatoren, wie zum Beispiel Natriumcromoglykat und Ketotifen; bronchiale Entzündungshemmer, wie zum Beispiel Nedocromilnatrium, Steroide, wie zum Beispiel Beclomethasondipropionat, Fluticason, Budesonid und Flunisolid; und Kombinationen derselben.

53. Pulverzuführungsvorrichtung nach Anspruch 52, **dadurch gekennzeichnet, dass** die Kombination von Pulvern ausgewählt wird aus Steroiden, wie zum Beispiel Beclomethasondipropionat, Fluticason, Budesonid und Flunisolid; und Kombinationen von β₂-Agonisten, wie zum Beispiel Formoterol und Salmeterol.

54. Pulverzuführungsvorrichtung nach Anspruch 28, **dadurch gekennzeichnet, dass** das Pulver systemisch wirksam ist.

55. Pulverzuführungsvorrichtung nach Anspruch 54, **dadurch gekennzeichnet, dass** das Pulver ausgewählt wird aus eiweißartigen Verbindungen und/oder Makromolekülen, wie zum Beispiel Hormone und Zwischenträger, wie zum Beispiel Insulin, menschliches Wachstumshormon, Leuprolid und Alphainterferon; Wachstumsfaktoren, Antikoagulanzien, Immunmodulatoren, Zytokine und Nukleinsäuren.

56. Pulverzuführungsvorrichtung nach Anspruch 28, **dadurch gekennzeichnet, dass** das Pulver eine Kombination ist, ausgewählt aus einem Pulver, wie durch Anspruch 52 definiert, und einem Pulver, wie durch Anspruch 53 definiert.

## Revendications

1. Système amplificateur d'air comprenant un jet de fluide amplificateur (3 ; 123 ; 502) muni d'une entrée de fluide (4 ; 124) et d'une sortie de fluide (5 ; 125 ; 503), la sortie de fluide (5 ; 125 ; 503) étant reliée à une buse de sortie (6 ; 114 ; 506) via un passage amplificateur (7), le passage amplificateur (7) étant relié aussi à une chambre de poudre (8 ; 130 ; 505), ladite chambre étant conçue pour un écoulement de poudre non laminaire, de telle sorte que le fluide se déplaçant depuis la sortie de fluide (5 ; 125 ; 503) du jet entraîne l'air extérieur et la poudre transformée en aérosol à travers la chambre de poudre (8 ; 130 ; 505) de sorte que l'air extérieur et la poudre transformée en aérosol se mélangent avec le fluide amplificateur dans le passage amplificateur (7) et le mélange amplifié sort par la buse de sortie (6 ; 114 ; 506) **caractérisé en ce que** la chambre de poudre (8 ; 130 ; 505) est une chambre annulaire qui est adaptée pour produire un écoulement non laminaire de la poudre.

2. Système amplificateur d'air selon la revendication 1 **caractérisé en ce que** le vide créé par la sortie du courant gazeux depuis le jet amplificateur (3 ; 123 ; 502) crée un vide dans la chambre de poudre (8 ; 130 ; 505) et un écoulement d'air d'entraînement au travers d'une poudre.

3. Système amplificateur d'air selon la revendication 2 **caractérisé en ce que** l'écoulement d'air d'entraînement est suffisant pour provoquer une désagglomération et/ou un entraînement puis une transformation de la poudre en aérosol.

4. Système amplificateur d'air selon la revendication 1 **caractérisé en ce que** le système comprend une chambre de poudre annulaire (8 ; 130 ; 505) et un jet de fluide axial (3 ; 123 ; 502).

5. Système amplificateur d'air selon la revendication 4 **caractérisé en ce que** la chambre de poudre (8 ; 130 ; 505) est sensiblement circonférentielle par rapport au corps du système amplificateur et le jet de fluide (3 ; 123 ; 502) est axial par rapport au corps.

6. Système amplificateur d'air selon la revendication 1 **caractérisé en ce que** la chambre de poudre annulaire (130) comprend une partie mâle (115) et une partie femelle (116).

7. Système amplificateur d'air selon la revendication 6 **caractérisé en ce que** l'extrémité de sortie (125) du jet de fluide (123) comprend un membre mâle tronconique (115) qui s'adapte dans une partie femelle (116) de la chambre de poudre (130).

8. Système amplificateur d'air selon la revendication 6 **caractérisé en ce que** la séparation entre les membres mâle (115) et femelle (116) (l'intervalle) est de 500 à 2 000 µm.

9. Système amplificateur d'air selon la revendication 8 **caractérisé en ce que** l'intervalle est d'environ 1 000 µm.

10. Système amplificateur d'air selon la revendication 9 **caractérisé en ce que** le diamètre du jet (123) est de 100 à 500 µm.

11. Système amplificateur d'air selon la revendication 10 **caractérisé en ce que** le diamètre du jet (123) est de 200 à 300 µm.

12. Système amplificateur d'air selon la revendication 11 **caractérisé en ce que** le diamètre du jet (123) est d'environ 250 µm.

13. Système amplificateur d'air selon la revendication 1 **caractérisé en ce que** le diamètre de la buse (6 ; 114 ; 506) est de 100 µm à 1 500 µm.

14. Système amplificateur d'air selon la revendication 13 **caractérisé en ce que** le diamètre de la buse (6 ; 114 ; 506) est de 400 µm à 1 200 µm.

15. Système amplificateur d'air selon la revendication 14 **caractérisé en ce que** le diamètre de la buse (6 ; 114 ; 506) est de 400 µm à 600 µm.

16. Système amplificateur d'air selon la revendication 15 **caractérisé en ce que** le diamètre de la buse (6 ; 114 ; 506) est d'environ 500 µm.

17. Système amplificateur d'air selon la revendication 1 **caractérisé en ce que** le diamètre de la buse (6 ; 114 ; 506) est supérieur au diamètre du jet (3 ; 123 ; 502).

18. Système amplificateur d'air selon la revendication 17 **caractérisé en ce que** le rapport du diamètre du jet (3 ; 123 ; 502) au diamètre de la buse (6 ; 114 ; 506) est de 1 : 8 à 1 : 2.

19. Système amplificateur d'air selon la revendication 18 **caractérisé en ce que** le rapport du diamètre du jet (3 ; 123 ; 502) au diamètre de la buse (6 ; 114 ; 506) est de 1 : 4 à 1 : 2.

20. Système amplificateur d'air selon la revendication 19 **caractérisé en ce que** le rapport du diamètre du jet (3 ; 123 ; 502) au diamètre de la buse (6 ; 114 ; 506) est 1 : 2.

21. Système amplificateur d'air selon la revendication 1 **caractérisé en ce que** la chambre (505) est munie d'un réservoir de poudre ou d'un membre doseur de poudre adjacent à une entrée (514) de la chambre de poudre.

22. Système amplificateur d'air selon la revendication 21 **caractérisé en ce que** le réservoir de poudre et/ou le membre doseur est contigu à la chambre de poudre (505).

23. Système amplificateur d'air selon la revendication 21 **caractérisé en ce que** la chambre de poudre (505) est reliée au réservoir de poudre et/ou au membre doseur par un ou plusieurs conduits (513).

24. Système amplificateur d'air selon la revendication 1 **caractérisé en ce que** le système comprend une pluralité de buses (414).

25. Système amplificateur d'air selon la revendication 1 **caractérisé en ce que** le système comprend une pluralité de jets de fluide (421).

26. Système amplificateur d'air selon la revendication 25 **caractérisé en ce que** la pluralité de jets (421) comprend une pluralité de jets qui se combinent.

27. Système amplificateur d'air selon la revendication 26 **caractérisé en ce que** chaque jet (421) est muni d'une entrée de poudre (413).

28. Système de délivrance de poudre qui comprend un passage de délivrance, un réservoir de poudre et/ou un membre doseur adapté pour présenter une dose mesurée de poudre au passage de délivrance **caractérisé en ce que** le dispositif de délivrance de poudre est muni d'un système amplificateur d'air selon la revendication 1.

29. Dispositif de délivrance de poudre selon la revendication 28 **caractérisé en ce que** le système amplificateur d'air crée un écoulement d'air entraîné au travers du réservoir de poudre et/ou du membre doseur.

30. Dispositif de délivrance de poudre selon la revendication 29 **caractérisé en ce que** l'air entraîné s'écoule au travers de la poudre qui est présentée directement depuis le réservoir ou depuis le membre doseur.

31. Dispositif de délivrance de poudre selon la revendication 30 **caractérisé en ce que** l'entrée d'air entraîné est positionnée en position adjacente à un premier côté du réservoir et/ou du membre doseur et un vide est créé en position adjacente à un second côté opposé du réservoir et/ou du membre doseur.

32. Dispositif de délivrance de poudre selon la revendication 29 **caractérisé en ce qu'**il est prévu un autre tube d'entrée qui est adapté pour introduire de l'air d'entraînement dans le réservoir/membre doseur.

33. Dispositif de délivrance de poudre selon la revendication 29 **caractérisé en ce que** l'écoulement d'air entraîné est suffisant pour désagglomérer la poudre et la transformer en un aérosol.

34. Dispositif de délivrance de poudre selon la revendication 28 **caractérisé en ce que** le dispositif est un dispositif d'inhalation.

35. Dispositif de délivrance de poudre selon la revendication 34 **caractérisé en ce que** le dispositif d'inhalation est un inhalateur de poudre sèche.

36. Dispositif de délivrance de poudre selon la revendication 34 **caractérisé en ce que** le système amplificateur est agencé pour réaliser le fonctionnement séparé, séquentiel ou simultané du ou des jets pour permettre la création d'une poudre transformée en aérosol qui coïncide avec l'inspiration d'un patient.

37. Dispositif de délivrance de poudre selon la revendication 28 **caractérisé en ce que** le fluide utilisé dans le jet de fluide est un gaz.

38. Dispositif de délivrance de poudre selon la revendication 37 **caractérisé en ce que** le gaz est produit par l'évaporation d'un propulseur volatil.

39. Dispositif de délivrance de poudre selon la revendication 28 **caractérisé en ce que** l'écoulement de fluide est produit par un moteur électrique.

40. Dispositif de délivrance de poudre selon la revendication 28 **caractérisé en ce que** l'écoulement de fluide est produit par un piston actionné manuellement.

41. Dispositif de délivrance de poudre selon la revendication 38 **caractérisé en ce que** le propulseur est un propulseur non-CFC.

42. Dispositif de délivrance de poudre selon la revendication 41 **caractérisé en ce que** le propulseur est un hydrofluoroalcane (HFA).

43. Dispositif de délivrance de poudre selon la revendication 42 **caractérisé en ce que** le propulseur est un fluoroalcane.

44. Dispositif de délivrance de poudre selon la revendication 43 **caractérisé en ce que** le fluoroalcane est un fluorométhane, un fluoroéthane ou un mélange de fluoroalcanes.

45. Dispositif de délivrance de poudre selon la revendication 44 **caractérisé en ce que** le fluoroalcane est choisi dans le groupe du trichlorofluorométhane, du dichlorodifluorométhane, du 1,2-dichlorotétrafluoroéthane, du trichlorotrifluoroéthane et du chloropentafluoroéthane.

46. Dispositif de délivrance de poudre selon la revendication 45 **caractérisé en ce que** le fluoroalcane est HFA 134 (1,1,1,2-tétrafluoroéthane).

47. Dispositif de délivrance de poudre selon la revendication 46 **caractérisé en ce que** le fluoroalcane est HFA 227.

48. Dispositif de délivrance de poudre selon la revendication 28 **caractérisé en ce que** le membre doseur comprend une poudre contenue dans un tambour et un support de tambour.

49. Dispositif de délivrance de poudre selon la revendication 28 **caractérisé en ce que** le membre doseur est une capsule.

50. Dispositif de délivrance de poudre selon la revendication 48 ou 49 **caractérisé en ce que** le dispositif est muni de moyens pour ouvrir le membre doseur.

51. Dispositif de délivrance de poudre selon la revendication 48 **caractérisé en ce que** le support de tambour fait partie intégrante du dispositif de délivrance.

52. Dispositif de délivrance de poudre selon la revendication 28 **caractérisé en ce que** la poudre est choisie dans le groupe de médicaments pour le traitement de l'asthme, de la COPD ou d'infections respiratoires comme les β₂-agonistes ; fénotérol, formotérol, pirbutérol, reprotérol, rimitérol, salbutamol, salmétérol et terbutaline ; les bêtastimulants non sélectifs comme l'isoprénaline ; les bronchodilatateurs de type xanthine ; la théophylline, l'aminophylline et le théophyllinate de choline ; les anticholinergiques comme le bromure d'ipratropium ; les stabilisants de mastocytes comme le cromoglycate de sodium et le kétotifen ; les agents anti-inflammatoires bronchiques comme le nédocromil sodium ; stéroïdes comme le dipropionate de beclométhasone, la fluticasone, le budésonide et le flunisolide ; et leurs combinaisons.

53. Dispositif de délivrance de poudre selon la revendication 52 **caractérisé en ce que** la combinaison de poudres est choisie parmi les stéroïdes, comme le dipropionate de beclométhasone, la fluticasone, le budésonide et le flunisolide ; et les combinaisons de β₂-agonistes comme le formotérol et le salmétérol.

54. Dispositif de délivrance de poudre selon la revendication 28 **caractérisé en ce que** la poudre est active systémiquement.

55. Dispositif de délivrance de poudre selon la revendication 54 **caractérisé en ce que** la poudre est choisie parmi les composés protéiniques et/ou les macromolécules, comme les hormones et les médiateurs, comme l'insuline, l'hormone de croissance humaine, le leuprolide et l'interféron alpha ; les facteurs de croissance, les anticoagulants, les immunomodulateurs, les cytokines et les acides nucléiques.

56. Dispositif de délivrance de poudre selon la revendication 28 **caractérisé en ce que** la poudre est une combinaison choisie parmi une poudre selon la revendication 52 et une poudre selon la revendication 53.
